Europäisches Patentamt

European Patent Office

Office européen des brevets

(11) Publication number: **0 454 024 A2**

## EUROPEAN PATENT APPLICATION

(21) Application number: 91106436.8

(22) Date of filing: 22.04.91

(51) Int. Cl.5: **C12P 23/00**, C12R 1/645, A23K 1/16, A23K 1/18

The applicant has filed a statement in accordance with Rule 28 (4) EPC (issue of a sample only to an expert). Accession number(s) of the deposit(s):NRRL Y-18549, 18550, 18551, 18552, 18553, 18554, 18555 & 18556.

(30) Priority: 23.04.90 US 513420

(43) Date of publication of application:
30.10.91 Bulletin 91/44

(84) Designated Contracting States:
BE DE DK GB NL SE

(71) Applicant: PHILLIPS PETROLEUM COMPANY
5th and Keeler
Bartlesville Oklahoma 74004(US)

(72) Inventor: Torregrossa, Robert Emile
3876 Telegraph Road
Elkton, MD 21921(US)
Inventor: Prevatt, William Dudley
4524 Barlow Drive
Bartlesville, OK 74006(US)

(74) Representative: Dost, Wolfgang, Dr. rer. nat.
Dipl.-Chem. et al
Patent- u. Rechtsanwälte Bardehle,
Pagenberg, Dost, Altenburg Frohwitter &
Partner Galileiplatz 1
W-8000 München 80(DE)

(54) A process for producing astaxanthin in phaffia rhodozyma.

(57) The present invention provides novel strains of *Phaffia rhodozyma*, which produce high levels of astaxanthin, a novel method for growing said *Phaffia rhodozyma*, and a novel method for providing the astaxanthin contained in *Phaffia rhodozyma* in a form suitable for use as a dietary pigment supplement.

EP 0 454 024 A2

Field of the Invention

The present invention relates to high productivity strains of *Phaffia rhodozyma* which provide astaxanthin in high yields, methods for the cultivation of *Phafia rhodozyma*, and methods for providing the astaxanthin produced in *Phaffia rhodozyma* in a form available as a dietary pigment supplement for fish, crustaceans, and birds.

Background of the Invention

Astaxanthin (3,3'-dihydroxy-$\beta$,$\beta$-carotene-4,4'-dione) is a oxycarotenoid pigment widely distributed in plants and animals. It is a predominant oxycarotenoid pigment in crustaceans, and salmonids. Astaxanthin is also found in algae, yeast (such as *Phaffia rhodozyma*) and birds.

In commercial aquaculture it is desirable to add astaxanthin to the diet of salmonids and crustaceans to impart the distinctive pink coloration found in indigenous salmonids, crustaceans and birds. Imparting this distinctive pink coloration to salmonids and crustaceans produced by commercial aquaculture is believed to be important in encouraging consumer acceptance of salmonids and crustaceans produced through aquaculture. Currently no economical source for natural astaxanthin exists.

One potential source of astaxanthin for aquacultural purposes is the yeast *Phaffia rhodozyma*. *Phaffia rhodozyma* has been recognized since its classification as a yeast species having a high astaxanthin content (~85% of its carotenoid pigment is astaxanthin, N.W. Miller, et al. Int. J. Syst. Bacteriol., Vol. 26, p. 286 (1976). Use of this yeast as a dietary supplement in salmonid and crustacean diets has also been explored by Eric A. Johnson and other researchers since the early 1980's.

The development of *Phaffia rhodozyma* as a commercial source of astaxanthin has been hampered by the absence of strains of *Phaffia rhodozyma* which have suitable growth characteristics and produce high yields of astaxanthin. Suitable growth characteristics for commercial strains of *Phaffia rhodozyma* would include growth rates which are comparable to wild strains of *Phaffia rhodozyma*, high strain stability, reduced tendency to foam, with few special requirements for vitamin or growth supplements. Additionally, commercial strains of *Phaffia rhodozyma* must also consistently produce high levels of astaxanthin. The strains of *Phaffia rhodozyma* currently available can produce from 30 to 2000 micrograms per gram of cell mass. Unfortunately, the high astaxanthin producing strains currently available exhibit extremely low growth rates (such as strain DBT 403 reported in WIPO application WO 88/08025) as compared to wild-type *Phaffia rhodozyma* strains which renders them unsuitable for commercial fermentation. Thus, it would be very advantageous for strains of *Phaffia rhodozyma* to be developed which produces high levels of astaxanthin (micrograms per gram of cell mass) while also exhibiting growth rates comparable to wild-type *Phaffia rhodozyma* (such as strain 67-210 which is publicly available from the United States Department of Agriculture, Northern Regional Research Center under accession number NRRL Y-10921).

Besides having suitable strains of *Phaffia rhodozyma* for commercial fermentation, methods for cultivating *Phaffia rhodozyma* need to be developed which maximize the yield of astaxanthin per cell mass. The literature presently advises culturing *Phaffia rhodozyma* on a growth limiting amount of sugar to avoid the metabolism of sugar to ethanol and acetaldehyde which occurs when excess amount of sugar is present. Of course, ethanol and acetaldehyde production in *Phaffia rhodozyma* cultivation is detrimental because of its high toxicity to *Phaffia rhodozyma* cells. Cells grown on limited sugar, however, produce lower levels of astaxanthin and release by products which cause excess foaming in the fermentation vessels. Foam severely reduces the usable fermentation mass per volume and must be controlled using mechanical or chemical means. Therefore, it would be a significant improvement over the art currently available to provide a method for the cultivating of *Phaffia rhodozyma* cells which solve some or all of these problems.

Finally, after the *Phaffia rhodozyma* cells have been harvested, a quick, economical method needs to be developed which provides the astaxanthin produced inside the cells as a readily available dietary pigment supplement for fish, crustaceans, and birds (animals unable to break open the *Phaffia* cell wall).

Thus it is an object of the present invention to provide strains of *Phaffia rhodozyma* which produce high levels of astaxanthin and are suitable for commercial fermentation.

It is also an object of the present invention to provide an improved method for the growth of *Phaffia rhodozyma* which produces higher yields of astaxanthin.

It is an additional object of the present invention to provide a method to render *Phaffia rhodozyma* cells which contain astaxanthin therein suitable as a dietary pigment supplement for fish, crustaceans, and birds.

Other aspects, objects and several advantages of this invention will be apparent from the instant specification.

## Summary of the Invention

In accordance with the present invention, we have discovered and isolated novel strains of *Phaffia rhodozyma* which produce in the range of from about 45 μg/l/hr to about 100 μg/l/hr of astaxanthin when grown under suitable growth conditions in a shake flask.

In accordance with a further embodiment of the present invention, we have additionally discovered and isolated novel strains of *Phaffia rhodozyma* which produce in the range of from about 400 μg/l/hr to about 962 μg/l/hr of astaxanthin when grown under suitable conditions in a fermentor.

In another embodiment of the present invention we have discovered a method for the cultivation of *Phaffia rhodozyma* cells which produces high yields of astaxanthin comprising:

(a) contacting *Phaffia rhodozyma* cells with a suitable nutrient media to form a fermentation broth;

(b) contacting said fermentation broth during cultivation with at least one suitable carbon-energy source and maintaining a measurable concentration of said at least one suitable carbon-energy source wherein the amount of at least one suitable carbon-energy source contacted with the fermentation broth does not result in the production of a growth inhibiting amount of ethanol by the *Phaffia rhodozyma* cells present in the fermentation broth; and

(c) cultivating said fermentation broth under pH, temperature, and aeration conditions to facilitate the growth of said *Phaffia rhodozyma* cells in the fermentation broth and.

In a further embodiment of the present invention we have discovered a method to provide *Phaffia rhodozyma* cells containing astaxanthin in a form wherein the astaxanthin is available to salmonids, crustaceans or birds as a dietary source of astaxanthin comprising:

(a) contacting *Phaffia rhodozyma* cells containing astaxanthin with an effective amount of a digestive enzyme preparation from the fungus *Trichoderma harzianum* capable of at least partially digesting the cell wall of *Phaffia rhodozyma*.

## Detailed Description of the Invention

Wild strains of *Phaffia rhodozyma* can produce between 30-800 μg astaxanthin/g yeast, depending on the growth conditions (Johnson, E. A. and Lewis, M. J., 1979. J. Gen. Microbiol. 115:173-183). While these levels of astaxanthin production are significant, to make commercial production of astaxanthin from *Phaffia rhodozyma* feasible significant improvements in the yields of astaxanthin must be obtained.

The astaxanthin productivity in *Phaffia rhodozyma* strains can be improved by a systematic strain development program consisting of treating the yeast cells with a mutagen and then screening the resultant mutant cells able to produce colonies which have higher concentrations of astaxanthin. Mutagens suitable for this process include but are not limited to ultraviolet radiation, nitrous acid, ethylmethanesulfonate (EMS), diethyl sulfate, l-methyl-l-nitro-nitrosoguanidine (nitrosoguanidine, NTG), S-bromouracil, 2-ominopurine, acridine orange, ethidium bromide and combinations of two or more thereof. These mutagens can be used to induce mutation in *Phaffia rhodozyma* following similar protocols to those developed for other yeast or bacteria, such as *Saccharomyces cerevisiae* or *Escherichia coli*. Currently preferred are yeast mutational protocols utilizing nitrosoguanadine or EMS. Suitable techniques for mutating yeasts such as *Phaffia rhodozyma*, are known to those skilled in the art including but not limited to the yeast mutation techniques discussed in Shermal, et al., Methods in Yeast Genetics, Cold Spring Harbor Laboratory, 1979, or Example I sections A-C. Screening can be accomplished by visual inspection, astaxanthin extraction assays, and fermentation studies of those colonies appearing to have improved astaxanthin levels. Strains derived from colonies of mutagenized *Phaffia rhodozyma* should also be screened to ensure that the strains will retain desirable characteristics during and after repeated fermentations.

Many rounds of mutagensis and screening are necessary to develop *Phaffia rhodozyma* strains which have significantly improved astaxanthin production levels and desirable growth characteristics. Unfortunately each round of mutagensis independently produced a new group of mutants which have a progressively lower probability of providing improved strains of *Phaffia rhodozyma*. Additionally each round of mutagensis increased the likelihood that undesirable mutations would occur causing fewer of the strains to demonstrate improved astaxanthin production and retain desirable growth characteristics. After multiple rounds of mutation several strains which appeared to be high astaxanthin producers on plate screening turned out to produce low levels of astaxanthin, unacceptable levels of foam, or slow growth rates during fermentation. The following strains were mutated with nitrosoquanidine or EMS or combinations thereof, and demonstrated significantly improved astaxanthin production levels, while retaining desirable fermentation

characteristics.

## TABLE I

| Phillips Culture Collection No. | NRRL No. | Astaxanthin Content[1] µg/g DCW[4] | Astaxanthin Productivity[2] µg/l/hr[3] |
|---|---|---|---|
| PC 8055* | Y-10291 | 530 | 43.2 |
| PC 8059[5] | Y-18549 | 726 | 79 |
| PC 8065[5] | Y-18550 | 720 | 56 |
| PC 8071[5] | Y-18554 | 977 | 58 |
| PC 8075[5] | Y-18555 | 1681 | 98 |
| PC 8077[5] | Y-18556 | 1345 | 47 |
| PC 8088[5] | Y-18551 | 1622 | 81 |
| PC 8091[5] | Y-18552 | 1988 | 52 |
| PC 8093[5] | Y-18553 | 1811 | 55 |

*The parent strain was 67-210, also known as PC 8055, which is deposited and accessible to the public from the United States Department of Agriculture, Agricultural Research Service, Northern Regional Research Center located in Peoria, Illinois under accession number NRRL Y-10921.

[1]Astaxanthin Content was determined by the method described in Example I.C.2.

[2]The productivity was determined by HPLC as described in Example I.

[3]The strains were grown in shake flasks as described in Example I C2. The strains were grown for 5 days (120 hours) then harvested and assayed.

[4]DCW is the abbreviation for dry cell weight. The dry cell weights were determined by taking a 25 ml sample of the broth and centrifuging the broth until a pellet of cells was formed. The supernatent was poured off and the pellet was resuspended in deionized water to provide a solution with a final volume of 25 ml. The resuspended solution was again centrifuged until a pellet of cells was formed. The supernatent was poured off and the pellet of cells was mixed with enough deionized water to form a pourable cell slurry. The slurry was placed in an aluminum tare pan. The centrifuge tube was also rinsed to remove any cell residue with approximately 5 ml of deionized water which was also added to the pan containing the cell slurry. The pan was placed in a drying oven at 80°C for 12 hours. At the end of the 12 hours the pan was weighted and the tare weight subtracted to give the dry cell weight.

[5]The isolated substantially pure strains of *Phaffia rhodozyma* with corresponding NRRL numbers have been deposited with the United States Department of Agriculture, Agricultural Research Service, Northern Regional Research Center, 1815 North University Street, Peoria, Illinois 61604, under the terms of the Budapest Treaty.

With the discovery of *Phaffia rhodozyma* strains PC 8059, PC 8065, PC 8071, PC 8075, PC 8077, PC 8088, PC 8091, and PC 8093 the increase in astaxanthin productivity is due to increased levels of astaxanthin production coupled with fast cell growth rate. The increased astaxanthin productivities of these strains under suitable growth conditions is in the range of from about 45 µg/l/hr to about 100 µg/l/hr of astaxanthin on a dry weight basis when cultivated in a shake flask. Preferably the astaxanthin productivity of these strains under the conditions described above will be in the range of from about 55 µg/l/hr to about

100 μg/l/hr on a dry weight basis and most preferably will range from about 78 μg/l/hr to about 100 μg/l/hr of astaxanthin on a dry weight basis. Suitable growth conditions in a shake flask are defined as the conditions necessary to provide the maximum specific growth rate for the *Phaffia rhodozyma* strain being cultivated in a shake flask which is being vigorously agitated after 5 days of growth. Suitable growth conditions for the *Phaffia rhodozyma* strains of the present invention in a shake flask include utilizing Rich Assay Growth Medium as defined in the Examples and cultivating the strain at between 20° C to 22° C with vigorous shaking.

Similarly these *Phaffia rhodozyma* strains also exhibit increased astaxanthin productivity under batch-fed fermentation conditions. The increase astaxanthin productivities of these strains under suitable growth conditions is in the range of from about 400 μg/l/hr to about 962 μg/l/hr of astaxanthin on a dry weight basis when cultivated in a batch-fed manner in a suitable fermentation vessel. Preferably the astaxanthin productivity of these strains under suitable growth conditions will be in the range of from 480 μg/l/hr to about 962 μg/l/hr of astaxanthin on a dry weight basis when cultivated under batch-fed conditions in a suitable fermentation vessel. Suitable growth conditions for batch-fed growth in a suitable fermentation vessel are defined as the conditions necessary to provide the maximum specific growth rate of the *Phaffia rhodozyma* strain being cultivated. Suitable growth conditions for the *Phaffia rhodozyma* strains of the present invention when cultivated in a batch-feed manner include innoculating the fermentation vessel with a fresh innoculum, into a suitable fermentation vessel (such as a 20 liter Bio Lafitte fermentation vessel) wherein the fermentation broth is the Bio Lafitte media described in the Examples, the pH is maintained between 4.5 and 5.5, the temperature is maintained between 19° C and 21° C, the dissolved oxygen content was maintained between 30% and 80% of saturation and a measurable glucose content in the range of from about 1 gram/liter to about 3 gram/liter was maintained until 24 hours before the fermentation was complete.

The currently preferred strain for the production of astaxanthin is strain PC 8059 NRRL Y-18549 because of its level of astaxanthin production and highly desirable growth characteristics.

## Fermentation

*Phaffia rhodozyma* is a relatively new organism for use in industrial fermentation. Several workers in the area of *Phaffia rhodozyma* fermentation have observed that alcohol or aldehydes will accumulate in levels toxic to *Phaffia* if an excess of a carbon-energy source in the form of sugar is provided. This has led these workers to suggest growing *Phaffia rhodozyma* cells under conditions where the amount of carbon-energy source provided limits growth conditions. However, *Phaffia rhodozyma* responds to carbon-energy source limitation by producing lower astaxanthin yields and releasing compounds which cause excessive foaming in the fermentation vessel. The presence of these foam causing compounds necessitates the use of antifoamants to avoid fermentation vessel overflows. Unfortunately the utilization of antifoamants can further reduce the per cell astaxanthin yields.

We, however, have discovered that by maintaining a measurable excess of at least one suitable carbon-energy source in the fermentation broth containing the aqueous *Phaffia rhodozyma* cells and nutrients that alcohol and aldehyde production can be easily controlled and foaming avoided. Additionally the presence of a measurable excess of at least one suitable carbon-energy source also results in increased cell growth rates and astaxanthin yields.

Particularly important in improving astaxanthin and cell yields is the maintenance of a measurable excess of at least one suitable carbon-energy source while the *Phaffia rhodozyma* cells are in the transition phase between innoculation and the logarithmic growth phase. Preferably the *Phaffia rhodozyma* cells will be contacted with a measurable excess of at least one suitable carbon-energy source from the transition phase through a substantial portion of the logarithmic growth phase.

The measurable excess of at least one suitable carbon-energy source provided should be an effective amount to avoid excessive foam formation during the fermentation of *Phaffia rhodozyma* and also not result in the generation of growth repressing or toxic levels of alcohol or aldehyde. Preferably the measurable excess of at least one carbon-energy source detectable in the fermentation broth consisting of the aqueous *Phaffia rhodozyma* cells and nutrients, will range from about 1.0 gram/liter to about 20.0 grams/liter and most preferably it will range from about 1 gram/liter to about 5 grams/liter. The amount of measurable excess of at least one suitable carbon-energy source in the fermentation broth should be controlled to avoid excess alcohol or aldehyde production. Preferably the amount of alcohol in the fermentation broth should range from about 0.0 grams/liter to about 3.0 grams/liter. Preferably the amount of aldehyde present in the fermentation broth will range from about 0.0 grams/liter to 0.1 grams/liter.

The fermentation of *Phaffia rhodozyma* can be conducted in a aqueous continuous or batch-fed manner, utilizing a variety of carbon-energy sources and/or nutrient sources. Suitable carbon-energy sources for growing *Phaffia rhodozyma* include but are not limited to the carbon-energy source selected from the group consisting of succinate, furamate, malate, pyruvate, glucose, sucrose, fructose, maltose, corn syrup, hydrolyzed starch, starch and combinations of any two or more thereof. Preferred carbon-energy sources for growing *Phaffia rhodozyma* are carbon-energy sources selected from the group consisting of succinate, glucose and combinations thereof. A suitable nutrient source or media for *Phaffia rhodozyma* would include at least one nitrogen source, at least one phosphate source, at least one source of minerals such as iron, copper, zinc, magnesium, manganese, calcium, and other trace elements, and vitamins (such as biotin, pantothenic acid and thiamine as required).

Suitable sources of at least one carbon-energy source and nutrients can be obtained from a variety of sources or may consist of a single source such as cane molasses. However, preferred are at least one carbon-energy source and/or nutrient sources which have a defined character. One carbon-energy source and/or nutrient composition which has proven effective is:

## Table II

### Carbon-Energy Source and Nutrients

#### Component per Liter of Water

| | |
|---|---|
| Carbon-energy Source | 10 - 100 (g/l) |
| $H_3PO_4$ (85%) | 0.16 - 2.7 (ml/l) |
| $CaSO_4 \cdot 2H_2O$ | 0.011 - 0.8 (g/l) |
| $K_2SO_4$ | 0.171 - 1.3 (g/l) |
| $MgSO_4 \cdot 7H_2O$ | 0.140 - 1.56 (g/l) |
| KOH | 0.047 - 0.35 (g/l) |
| Biotin | 0.006 - 0.044 (mg/l) |
| Thiamine | 0.12 - 9.8 (mg/l) |
| [1]Yeast extracts | 1.2 - 6.0 (g/l) |
| [2]Minerals and Trace metals | 0.118 - 9.8 (ml/l) |

[1]Yeast extract is Amberex[TM] 1003 which is available from and a trademark of Universal Foods Corporation, Milwaukee, Wisconsin.

[2]Minerals and trace metals are $FeSO_4 \cdot 7H_2O$ 65.0 g/l, $CuSO_4 \cdot 5H_2O$ 6.0 g/l, $ZnSO_4 \cdot 7H_2O$ 20 g/l, $MnSO_4$ 3.0 g/l and $H_2SO_4$ 5.0 ml/l

The yeast extracts utilized in the present invention include but are not limited to yeast extracts selected from the group consisting of AmberexTM 1003 and BactoTM Yeast Extract (Difco Laboratories Incorporated). Alternatively corn steep liquor could be used to replace yeast extracts as a source of nitrogen.

Trace metals utilized in the present invention are those trace metals generally utilized in yeast growth provided in an amount sufficient to not limit the growth rate or astaxanthin production of *Phaffia rhodozyma* which include but are not limited to trace metals selected from the group consisting of cobalt, molybdenum, iron, copper, zinc, and manganese.

The fermentation temperature should generally range from about 18°C to about 22°C and preferably should be about 20°C.

The dissolved oxygen content in the fermentation vessel where the fermentation is conducted in a batch-fed manner may range from about 10% to about 80% of saturation and preferable will range from about 30% to about 60% of saturation. The dissolved oxygen content in a continuous fermentation should range from about 70% to about 100% of saturation and preferably be in the range of from about 70% to about 80% of saturation. The pH at which the *Phaffia rhodozyma* cells are cultivated should range from about 3.0 to about 5.5 and preferably the pH will range from about 4.5 to about 5.4.

After the fermentation broth containing the *Phaffia rhodozyma* cells has reached a desired cell density or astaxanthin content, the cell mass may be harvested. It is preferred that the *Phaffia rhodozyma* culture be held in a stationary phase for from the range of from about 4 to about 24 hours and most preferably in the range of from about 8 to about 12 hours to increase the astaxanthin yield.

However, *Phaffia rhodozyma* should not be maintained for extended periods of time in a stationary phase because the *Phaffia rhodozyma* cells will form thick cell walls which will be detrimental to providing the astaxanthin contained in those cells in a form suitable for use as a dietary pigment supplement.

Cell Breakage

Salmonids, crustaceans and birds cannot utilize astaxanthin from unbroken *Phaffia rhodozyma* cells. To utilize *Phaffia rhodozyma* as a dietary source of astaxanthin the cell walls of *Phaffia rhodozyma* must be disrupted by physical, chemical, mechanical or enzymatic means. *Phaffia rhodozyma* cell walls are very resistant to normal lysis protocols. For example, bead milling will only release ~40% of the astaxanthin present in *Phaffia rhodozyma* cells after three passes through a bead mill (more passes through a bead mill will not substantially increase the release of astaxanthin). A Gualin Press will release ~95% of the astaxanthin present in *Phaffia rhodozyma* but only after three passes through the Gualin Press (which is a time consuming and requires a significant capital expenditure). Enzymatic lysis of *Phaffia rhodozyma* also had not proven to be economically or effective in releasing astaxanthin from *Phaffia rhodozyma* until the disccvery of the present invention.

We have discovered that an effective amount of a digestive enzyme preparation from the fungus *Trichoderma harzianum* is capable of of digesting the cell wall of *Phaffia rhodozyma*. This results in an almost complete availability of the astaxanthin present in *Phaffia rhodozyma* as determined by comparison to acetone extract which is described in Example I.C.2. Table 7 in Example III compares MutanaseTM - (Trademark of Novo Enzymes) a *Trichoderma harzianium* digestive extract, against several other commercial enzymatic extracts. Table 7 demonstrates that in minute quantities the *Trichoderma harzianium* digest enzyme preparation (MutanaseTM) released substantially all the astaxanthin contained in *Phaffia rhodozyma*.

Suitable strains of *Trichoderma harzianium* are publicly available from the American Type Culture Collection, 12301 Parklawn Drive, Rockville, Maryland (such as ATCC-64986). These strains may be cultured and stimulated to produce digestive enzymes by submerged culture fermentation as is known by those skilled in the art. One suitable source of *Trichoderma harzianium* digest enzyme preparations is Novo Enzymes SP-299-Mutanase.

*Phaffia rhodozyma* cells containing astaxanthin should be treated with an effective amount of *Trichoderma harzianium* digestive enzyme preparation to result in the availability of substantially all the astaxanthin contained therein. Generally the amount of digestive enzyme preparation per 100 grams/liter of aqueous *Phaffia rhodozyma* will be dependent on the temperature, pH and condition of the *Phaffia rhodozyma* cells employed, as a guideline it is recommended the amount of *Trichoderma harzianium* utilized that range from about 0.2 units to about 10.0 units of *Trichoderma harzianium* digestive enzyme preparation be utilized per 100 grams/liter. A unit is defined as the amount of *Trichoderma harzianium* digestive enzyme which provide the equivalent amount of released astaxanthin as the acetone extraction described in Example I.C.2, on a sample of aqueous *Phaffia rhodozyma* with a density of 100 grams/liter removed while in a logarithmic growth phase, when the digestive enzyme is contacted with the *Phaffia rhodozyma* cells at 22° C, and pH 4.5 and allowed to incubate for 24 hours.

Temperature at which *Phaffia rhodozyma* cells are contacted with the digestive enzyme preparation may be any temperature which allows the digestive enzyme preparation to digest *Phaffia rhodozyma* cell walls. Generally temperatures should range from about 0° C to about 60° C. Preferred for the practice of this invention are temperatures in the range of from about 20° C to about 30° C.

The pH at which *Phaffia rhodozyma* cells are contacted with the digestive enzyme preparation may be any suitable pH which permits the digestive enzyme preparation to digest *Phaffia rhodozyma* cell walls. Generally the pH at which *Phaffia rhodozyma* cells are contacted with the digest enzyme preparation should range of from about pH 4.0 to about pH 5.5 and preferably be in the range of from about pH 4.5 to about pH 5.0.

*Phaffia rhodozyma* cells containing astaxanthin may be contacted with the digestive enzyme preparation derived from *Trichoderma harzianium* at any time during the life cycle of *Phaffia rhodozyma*. However, it is preferred that the *Phaffia rhodozyma* cells be contacted with the digestive enzyme preparation as soon as possible after the *Phaffia rhodozyma* cells have been in a logarithmic growth phase, preferrably in the range of from about 0 hours to about 72 hours after a logarithmic growth phase and most preferrably in the range of from about 0 hours to about 24 hours.

The mixing of an aqueous suspension of *Phaffia rhodozyma* cells and the *Trichoderma harzianium* digestive enzyme preparation may be accomplished by any suitable means mixing is generally accomplished by contacting a dried digestive enzyme preparation into an aqueous *Phaffia rhodozyma* fermentation broth or aqueous cell suspension and admixing said dry digestive enzyme preparation into solution.

The digestive enzyme preparation derived from *Trichoderma harzianium* may be contacted with *Phaffia rhodozyma* cells which contain astaxanthin for an amount of time effective to result in the substantial release of astaxanthin present in the *Phaffia rhodozyma* cells as compared to acetone extraction described in Example I. The amount of time depends on the cell concentration, pH, temperature and units of digestive enzyme preparation utilized. Generally the time of contacting the *Phaffia rhodozyma* cells with the digestive enzyme preparation derived from *Trichoderma harzianium* should be in the range of about 12 hours to about 24 hours, preferable the time of contacting will be about 24 hours.

Drying of *Phaffia rhodozyma* Cells

The *Phaffia rhodozyma* cells after having been broken or digested in a manner which renders the astaxanthin contained therein available for use as a dietary pigment supplement can be dried. Drying may be performed using a fluidized bed drier, drum drier or spray drier. Spray drying is presently preferred because of the short exposure to high temperatures which could oxidize/decompose the astaxanthin present.

Spray drying should be performed on a slurry of broken or digested *Phaffia rhodozyma* cells. If the slurry is formed on *Phaffia rhodozyma* cells which have been digested with a digestive enzyme preparation, the slurry should be washed to remove any residues on the cell surfaces which would tend to make the cells sticky. Washing the cell slurry may be accomplished by any suitable technique known to those skilled in the art. The slurry may be optionally mixed with antioxidants such as butyl hydroxytoluene (BHT), butyl hydroxyanisol (BHA), or ascorbate or emulsifiers such as sorbital monostearate. The slurry should then be provided to the spray drier inlet which should have a temperature range from about 425°F to about 525°F and preferably from about 440°F to about 510°F. The critical parameter which should be monitored to control input rate of the slurry is the outlet temperature of the spray drier should be maintained from about 160°F to about 205°F and preferably from about 170°F to about 190°F. After drying the resultant product will be a powdery yeast material which may be recovered by any suitable means such as a cyclone and further handled for use in feed, storage or shipping.

The following non-limiting examples are provided to further illustrate the practice of the present invention.

Examples

Strains

| | | |
|---|---|---|
| *Phaffia rhodozyma* | PC 8055 | NRRL Y-10921 |
| *Phaffia rhodozyma* | PC 8059 | NRRL Y-18549 |
| *Phaffia rhodozyma* | PC 8065 | NRRL Y-18550 |
| *Phaffia rhodozyma* | PC 8088 | NRRL Y-18551 |
| *Phaffia rhodozyma* | PC 8091 | NRRL Y-18552 |
| *Phaffia rhodozyma* | PC 8093 | NRRL Y-18553 |
| *Phaffia rhodozyma* | PC 8071 | NRRL Y-18554 |
| *Phaffia rhodozyma* | PC 8075 | NRRL Y-18555 |
| *Phaffia rhodozyma* | PC 8077 | NRRL Y-18556 |

Media, Buffers and Solutions

YEPD

1% Bacto yeast extract
2% Bacto peptone
2% Dextrose

Citrate buffer    pH 5.5    .1M

Phosphate buffer    pH 4.5    .1M

20% polyethylene glycol (PEG - 3350)

| Rich Assay Growth Medium | | |
|---|---|---|
| | Bacto Yeast Extract | 9.0 g/l |
| | Difco Malt Extract | 6.0 g/l |
| | Dextrose | 10.0 g/l |
| | $KH_2PO_4$ | 15.0 g/l |
| | $K_2HPO_4$ | 1.0 g/l |
| | $MgSO_4 \cdot 7H_2O$ | 0.5 g/l |
| | $CaSO_4 \cdot 2H_2O$ | 0.04 g/l |
| | $(NH_4)_2SO_4$ | 3.0 g/l |
| | $FeSO_4 \cdot 7H_2O$ | 16.25 mg/l |

9

|  | | | |
|---|---|---|---|
| | $CuSO_4 \cdot 5H_2O$ | 1.5 | mg/1 |
| | $ZnSO_4 \cdot 7H_2O$ | 5.0 | mg/1 |
| | $MnSO_4 \cdot H_2O$ | 0.75 | mg/1 |
| | Biotin | 0.05 | mg/1 |
| | Thiamine | 1.00 | mg/1 |
| | Water | 1 L | |

pH to 5.0 with NaOH or $H_3PO_4$

| Modified YMA Medium | | | |
|---|---|---|---|
| | Bacto Yeast Extract | 6 | g/1 |
| | Difco Malt Extract | 6 | g/1 |
| | Dextrose | 20 | g/1 |
| | Agar | 20 | g/1 |
| | Water | 1.0 | L |

| Minimal Fermenter Feed/FM-21 | | | |
|---|---|---|---|
| | 10% carbon source | | |
| | $H_3PO_4$ 85% | 3.5 | ml/1 |
| | $CaSO_4 \cdot 2H_2O$ | 0.15 | g/1 |
| | $K_2SO_4$ | 2.38 | g/1 |
| | $MgSO_4 7H_2O$ | 1.95 | g/1 |
| | KOH | 0.65 | g/1 |
| | YTM-4 | 1 | ml/1 |
| | Water | 1 L | |

pH to 5.0 with $NH_3$ or $NH_4OH$

| IM3 Salts Medium | | | |
|---|---|---|---|
| | $KH_2PO_4$ | 15.0 | g/1 |
| | $K_2HPO_4$ | 1.0 | g/1 |
| | $MgSO_4 \cdot 7H_2O$ | 0.50 | g/1 |
| | $CaSO_4 \cdot 2H_2O$ | 0.04 | g/1 |
| | $(NH_4)_2SO_4$ | 3.00 | g/1 |
| | Biotin | 0.05 | mg/1 |
| | Water | 1 L | |
| | pH to 5.4 | | |

| Bio Lafitte Media | | | |
|---|---|---|---|
| | $H_3PO_4$ (85%) | 14.5 | ml/1 |

| | |
|---|---|
| $CaSO_4 \cdot 2H_2O$ | 0.60 g/l |
| $K_2SO_4$ | 9.12 g/l |
| $MgSO_4 \cdot 7H_2O$ | 7.60 g/l |
| KOH | 2.60 g/l |
| glucose | 40 g/l |
| yeast extract | 20 g/l |
| trace metals | 4.0 ml/l |
| biotin | 8 mg/l |
| thiamine | 8 mg/l |
| STRUKTOL J867 or MAZU DF 37C | 12 drops/l |
| Water | 1 L |
| pH to 5.0 with $NH_3$ | |

[1]trace metals contain (YTM-4):

| | |
|---|---|
| $FeSO_4 \cdot 7H_2O$ | 16.25 g/250 ml |
| $CuSO_4 \cdot 5H_2O$ | 1.50 g/250 ml |
| $ZnSO_4 \cdot 7H_2O$ | 5.00 g/250 ml |
| $MnSO_4 \cdot H_2O$ | 0.75 g/250 ml |
| $H_2SO_4$ | 1.25 g/250 ml |
| Water | 1 L |

Vitamin Mix
US F&WS No. 30

| | |
|---|---|
| Vitamin A | 1,653,450 I.U./Kg |
| Vitamin $D_3$ | 110,230 I.U./Kg |
| Vitamin E | 88,184 I.U./Kg |
| Vitamin $B_{12}$ | 5.5 mg/Kg |
| Riboflavin | 13,228 mg/Kg |
| Niacin | 55,115 mg/Kg |
| d-Pantothenic Acid | 26,455 mg/Kg |
| Menadione | 902 mg/Kg |
| Folic Acid | 2,205 mg/Kg |
| Pyridoxine | 6,349 mg/Kg |
| Thiamine | 8,102 mg/Kg |
| d-Biotin | 88 mg/Kg |

Mineral Mix

| US F&WS No. 3 | | |
|---|---|---|
| Zinc | 34,000 | mg/lb |
| Manganese | 9,100 | mg/lb |
| Copper | 700 | mg/lb |
| Iodine | 4,500 | mg/lb |

Example I

Development of Improved Strains of *Phaffia rhodozyma*

The following protocols were used to develop strains PC 8059, PC 8065, PC 8075, PC 8077, PC 8088, PC 8091, and PC 8093.

A. NTG Mutagenesis of *Phaffia rhodozyma*

*Phaffia rhodozyma* was subcultured into 50 ml of YEPD in a 250 ml Ehrlenmeyer flask and incubated on a shaker for 48 hr at 20°C. The cells were then centrifuged in sterile 30 ml tubes at 12,000 xg at 4°C for 10 min and washed two times with 30 ml of sterile, pH 5.5 citrate buffer. The cell pellet was resuspended in 27 ml of sterile pH 5.5 citrate buffer and 3 ml of 1mg/ml NTG (in pH 5.5 citrate buffer) was added. The cell suspension was then incubated for 15 min at room temperature without shaking. The suspension was centrifuged at 12,000 x g at 4°C for 5 min and then washed two times with sterile deionized water. The cells were then resuspended in 30 ml of YEPD in a 250 ml Ehrlenmeyer flask and incubated on a shaker for 20 min at 20°C. Using sterile 0.1M MgSO₄, the cells were diluted 1/100 and 0.1 ml was plated on 20 YEPD plates. These plates were then incubated at 20°C for ten days. After ten days the plates were counted and scored for mutants.

B. EMS Mutagenesis of *Phaffia rhodozyma*

*Phaffia rhodozyma* was subcultured into 50 ml of YEPD in a 250 ml Ehrlenmeyer flask and incubated on a shaker at 20°C for 2 days. The cells were centrifuged in sterile 30 ml tubes at 12,000 xg at 4°C for 10 min. The pellet was then washed two times with 30 mls of sterile, pH 5.5 citrate buffer. 0.9 ml of the cell suspension was placed into each of 13 sterile 1.5 ml conical bottom, polypropylene microcentrifuge tubes and 75 µl of EMS (ethylmethanesulfonate) was added to each tube. One tube was immediately removed, centrifuged with a microcentrifuge to pellet the cells, washed two times with 1.0 ml of sterile deionized water, resuspended in 1.0 ml of sterile deionized water and kept on ice until time for dilution. This step was repeated every 5 min for subsequent tubes (up to 60 min total exposure to EMS). All tubes were then diluted $10^2$, $10^3$, $10^4$, $10^5$, $10^6$, and $10^7$ times in sterile 0.1M MgSO₄ and 0.1 ml of each dilution was plated on 2 YEPD plates. All plates were incubated at 20°C for 10 days. After 10 days the plates were counted and scored for mutants.

C. Screening Process

1. Plate Assay of Astaxanthin

a. *Phaffiarhodozyma* Growth on Plates

Modified YMA plates were streaked with cultures to be tested and incubated at 20-22°C aerobically. After four days, using a sterile applicator stick or loop, one or two large colonies were scraped off (0.1-0.2 grams net weight) from each Modified YMA culture plate. The cells were resuspended in 1.0 ml of deionized, distilled water and placed in a 2.0 ml conical bottom polypropylene microcentrifuge tube.

b. Acetone Extraction of Astaxanthin

0.1 ml of each cell suspension was pipetted into 9.9 ml of deionized, distilled water, and the absorption was measured at 600 nm, to determine the cell concentration (in grams of washed cell dry weight per liter) with this method, an original cell concentration of 15 g/L has at a wavelength of 600nm (using a 1 cm light path) an absorption of 0.550. The measurement is linear over an absorption range of 0.000 to 1.000, or 0 to 27 gm/L of cells.

The original cell suspension (0.9 ml volume) was then pelleted in a microcentrifuge at 14,000 xg at 4°C and the supernatant was decanted. Acetone was added to provide a total volume of 1.0 ml. 0.25 grams of 450-500 micron glass beads were added and vigorously agitated for at least 10 min. total time (with periodic cooling intervals on ice). After 10 minutes the agitation should have resulted in the breakage of substantially all the *Phaffia rhodozyma* cells present, which should be verified microscopically. If the breakage was not substantially complete the bead milling process should be repeated until substantial breakage does occur. The resulting cell debris and glass beads were pelleted in a microcentrifuge at 14,000 xg and 4°C. 0.5 ml of supernatant was diluted to 1.5 ml with acetone. The absorption was then measured at 478 nm.

Astaxanthin concentration was calculated as follows:

assume 1 $\mu$g/l of pure astaxanthin

has an $A_{478} = 160 \times 10^{-6}$ with a 1 cm light path

Total astaxanthin concentration of the original 1.0 ml cell suspension is calculated as follows:

$$[\text{astaxanthin}] \text{ in } \mu g/l = \frac{A_{478}}{b \times 160 \times 10^{-6}} \times \text{dilution factor}$$

where b = length of light path in cm (usually 1) and

$$\text{dilution factor} = \frac{1.5 \text{ ml}}{0.5 \text{ ml}} \times \frac{1.0 \text{ ml}}{0.9 \text{ ml}} = 3.33$$

Cellular astaxanthin concentration is then calculated as follows:

$$[\text{astaxanthin}] \text{ in } \mu g/g \text{ of cells} =$$

$$\frac{\text{astaxanthin concentration } (\mu g/l)}{\text{cell concentration } (g/l)}$$

## 2. Shake Flask Assay of Astaxanthin

### a. *Phaffiarhodozyma* Growth in Shake Flask

50 ml of Rich Assay Growth Medium was inoculated with a loop full of *Phaffia* culture from a 7 day old plate culture in a 250 ml Ehrlenmeyer flask. The inoculated flask was then incubated at 20-22°C with vigorous shaking. After five days each of the cultures were harvested.

### b. Acetone Extraction of Astaxanthin

Each culture was harvested by centrifugation at 12,000 xg for 10 min at 4°C to pellet the cells. Each pellet was washed 2x with 100 ml of deionized, distilled water and finally resuspended in 100 ml of deionized, distilled water. 0.1 ml of each cell suspension was pelleted into 9.9 ml of deionized, distilled water and the absorption was measured at 600 nm to determine the cell concentration. Using this method, an original cell concentration of 15 g/l has at a wavelength of 600 nm (using a 1 cm light path) an absorption of 0.550. The measurement is linear over an absorption range of 0.000 to 1.000 or 0 to 27 gm/l of cells.

10 ml of each original cell suspension was pelleted and resuspended in 1.0 ml of deionized, distilled water and transferred to a 2.0 ml conical bottom, polypropylene microcentrifuge tube. The cells were pelleted in the tube by centrifugation at 14,000 x g and 4°C. The supernatant was then decanted and acetone was added to provide a 1.0 ml final volume.

0.25 grams of 450-500 micron glass beads were added to the sample and vigorously agitated for at least 10 min total time (with periodic intervals for cooling on ice). After 10 min. the agitation should have resulted in the breakage of substantially all the *Phaffia rhodozyma* cells present, which should be verified microscopically. If the breakage was not substantially complete the bead milling process should be repeated until substantial breakage did occur. The glass beads and resulting cell debris were pelleted by centrifugation at 14,000 x g and 4°C. 0.5 ml of the supernatant was removed and 1.0 ml of acetone was added. The absorption was measured at 478 nm.

Assume 1μg/l of pure astaxanthin has an $A_{478} = 160 \times 10^{-6}$ with a 1 cm light path

Total astaxanthin concentration of the original 100 ml cell suspension:

$$[\text{astaxanthin}] \text{ in } \mu/g/1 = \frac{A_{478}}{b \times 160 \times 10^6} \times \text{dilution factor}$$

where b = length of light path in cm (usually) and dilution factor

$$= \frac{1.5 \text{ ml}}{0.5 \text{ ml}} \times \frac{10 \text{ ml}}{1 \text{ ml}} = 0.3$$

Cellular astaxanthin concentration was then calculated as follows:

$$[\text{astaxanthin}] \text{ in } \mu g/g \text{ of cells } =$$

$$\frac{\text{astaxanthin concentration (μg/1)}}{\text{cell concentrations (g/1)}}$$

$$[\text{astaxanthin}] \text{ in } \mu g/g \text{ DCW } =$$

$$\frac{\text{astaxanthin concentration (μg/R)}}{\text{cell concentration (g/1) DCW}}$$

Dry cell weight (DCW) was determined by taking a 25 ml sample of the broth and centrifuging the broth until a pellet of cells was formed. The supernatent was poured off and the pellet was resuspended in deionized water to provide a cell suspension with a final volume of 25 ml. The cell suspension was again centrifuged until a pellet of cells was formed. The supernatent was poured off and the pellet of cells was mixed with enough deionized water to form a pourable cell slurry. The slurry was placed in an aluminum tare pan. The centrifuge tube was also rinsed to remove any cell residue with approximately 5 ml of deionized water which was also added to the pan containing the cell slurry. The pan was placed in a drying oven at 80°C for 12 hours. At the end of the 12 hours the pan was weighted and the tare weight subtracted to give the dry cell weight.

3. HPLC Assay of Astaxanthin

Five 0.2 ml samples of yeast broth containing 0.05 grams per ml of yeast as measured at 600 nm were separately placed in five 1.5 ml conical bottomed centrifuge tubes. Cell concentration was calculated using an original cell concentration of 15 grams per liter and having an absorption of 0.550 (using a 1 cm lightpath, assuming the measurement is linear between a 0 to 15 gram per liter concentration). The samples were then centrifuged in a microcentrifuge at 14,000xg for 5 minutes at 4°C to pellet the cells. 1.0 ml of acetone was added to each of the samples as well as 0.25 grams of glass beads (450-500 micron glass beads washed in 10% phosphoric acid rinsed in distilled dionized water and dried at 80°C). The tubes were then vortexed vigorously for 2 minutes at 4°C and centrifuged in the microcentrifuge at the 14,000 g for 10 minutes at 4°C to pellet the glass beads and cell debris. The supernatant was decanted from all the tubes into a 6 dram glass vial. The addition of acetone and vortexing followed by centrifuging were repeated until the supernatant produced by this process was colorless. The supernatant was then combined and dried in a hood in darkness under a nitrogen atmosphere at room temperature. The pigment which was left after the drying process was then mixed with exactly 5 mls of methanol and allowed to redissolve for 30 minutes. The methanol pigment solution was then filtered through a 0.2 micron filter. A 100 microliter sample of the pigment solution was then run in a Waters HPLC. The HPLC was run isocratically, with methanol as a solvent, a 2 ml per minute flow rate and the column was maintained at room temperature. The detector was set at 478 nm. The column was run for 10 minutes. The asaxthanin retention was approximately 2.6 to 2.7 minutes. Sample peak areas were calculated by a Hewlett Packard 3390A Integrater. A Waters radial-PAK C18 column (P-N 86342) was the column utilized in the HPLC.

Cellular astaxanthin concentration was then calculated as follows:

$$\text{[astaxanthin] in } \mu g/g \text{ of cells} =$$

$$\frac{\text{astaxanthin concentration } (\mu g/l)}{\text{cell concentrations } (g/l)}$$

$$\text{[astaxanthin] in } \mu g/g \text{ DCW} =$$

$$\frac{\text{astaxanthin concentration } (\mu g/l)}{\text{cell concentrations } (g/l) \text{ DCW}}$$

DCW is the abbreviation for dry cell weight. The dry cell weights were determined by taking a 25 ml sample of the broth and centrifuging the broth until a pellet of cells was formed. The supernatent was poured off and the pellet was resuspended in deionized water to provide a solution with a final volume of 25 ml. The resuspended solution was again centrifuged until a pellet of cells was formed. The supernatent was poured off and the pellet of cells was mixed with enough deionized water to form a pourable cell slurry. The slurry was placed in an aluminum tare pan. The centrifuge tube was also rinsed to remove any cell residue with approximately 5 ml of deionized water which was also added to the pan containing the cell slurry. The pan was placed in a drying oven at 80°C for 12 hours. At the end of the 12 hours the pan was weighted and the tare weight subtracted to give the dry cell weight.

The standard was obtained by dissolving 5 mg of crystalline astaxanthin in 100 ml of methanol. To calculate the exact concentration the absorption was measured at 478 nm. The extinction coefficient for a 1% solution at 1 cm was determined to be 2,350. The purity of the standard solution was verified by running HPLC analysis on several dilutions. Aliquots of the standard may be stored for up to 4 weeks in a -80°C freezer in the dark.

D. Development of Strains

*Phaffia rhodozyma* mutants were developed from the parent strain PC 8055 (NRRL Y-10921) using either NTG or EMS mutagenesis as detailed in Examples IA and IB. The following flow chart illustrates the genetic history of each mutant strain. The numbers on the left hand column indicate the generation. The letters EMS and NTG represent the type of mutagensis performed to derive the next listed generation. Each generation utilized the plate and shake flask screenings as well as the astaxanthin extraction with acetone to screen hundreds of progeny before the next round of mutagensis was performed.

## Table III

### Flow Chart of Mutant Development

```
                              PC8055
                                |
                                | NTG
                                v
                              PC8056
                                |
                                | NTG
                                v
                              PC8057
                                |
                                | NTG
                                v
      EMS  ┌─────────────────PC8058─────────────────┐  NTG
           |                    |                    |
           |                    | NTG                |
           v                    v                    v
        PC8059 ┌─────────────PC8065──────────┐    PC8062
           |   |                |             | NTG
           |NTG|                |NTG          |
           v   |                v          ┌──┴──┐
        PC8088 |             PC8081        |     |
           |                    |          |     |
           |NTG                 |NTG       v     v
           v                    v       PC8067 PC8070
        PC8089              PC8082               |
           |                                     |NTG
           |NTG                                  v
        ┌──┴──┐                               PC8071
        |     |                                  |
        v     v                                  |NTG
     PC8091 PC8093                               v
                                             PC8075
                                                |
                                                |NTG
                                                v
                                             PC8077
```

### Example II

A. High Cell Density Fermentation of *Phaffia rhodozyma.*

This example demonstrates that *Phaffia rhodozyma* may be grown under high cell density in a continuous fermentation process using the Phillips Petroleum Company High Cell Density fermentation process disclosed in U.S. 4,617,274 (and herein incorporated by reference), Phaffia rhodozyma PC 8057 was grown using the following conditions:

1. growth temperature was 22°C,
2. growth pH range was 4.5 to 5.0, using ammonia and phosphoric acid for pH control,
3. dissolved oxygen was maintained at greater than 75% saturation through increased agitation, supplementation of the airstream with oxygen, and/or increased fermentor vessel pressure,
4. growth medium consisted of FM-21 supplemented with 0.05 mg/l biotin, 1 mg/l thiamine, and 0.1% yeast extract,

5. chemostat dilution rates were maintained at 0.054 to 0.057 volumes per hour.

After fermentation, the chemostat effluent was held for 72 hours at room temperature but with no aeration, and pH controlled between 4.5 and 5.0 to allow the cellular astaxanthin content to increase to maximum levels. The results are summarized in Table 4.

TABLE 4

| DAY | % Glucose in Feed | Dilution Rate | pH | T | %DO | RPM | DRY CELL g/l | ASTAXANTHIN µg/l | PPM |
|---|---|---|---|---|---|---|---|---|---|
| 1 | 2 | | 4.7 | 21.5 | 100 | 300 | ND | ND | ND |
| 2 | 2 | | 4.7 | 21.5 | 100 | 300 | ND | ND | ND |
| 3 | 10 | .053 | 4.8 | 21.5 | 68 | 300 | 26.7 | 13969 | 523 |
| 4 | 10 | .059 | 4.7 | 21.6 | 71 | 300 | 23.7 | 12000 | 506 |
| 5 | 10 | .026 | 4.7 | 21.5 | 59 | 300 | ND | ND | ND |
| 6 | 10 | .043 | 4.7 | 21.4 | 45 | 300 | 36.6 | 18656 | 510 |
| 7 | 10 | .037 | 4.7 | 21.5 | 65 | 300 | 37.4 | 21338 | 571 |
| 8 | 10 | .037 | 4.7 | 21.6 | 100 | 300 | 37.5 | 23438 | 625 |
| 9 | 10 | .077 | 4.6 | 21.7 | 59 | 300 | 38.7 | 19500 | 504 |
| 10 | 10 | .050 | 4.6 | 21.6 | 51 | 300 | 39.2 | 20850 | 532 |
| 11 | 10 | .037 | 4.7 | 21.9 | 30 | 700 | ND | ND | ND |
| 12 | 20 | .037 | 4.6 | 21.8 | 18 | 700 | ND | ND | ND |
| 13 | 20 | .030 | 4.6 | 21.8 | 88 | 700 | 75.9 | 38250 | 504 |
| 14 | 20 | .042 | 4.6 | 22.0 | 100 | 1000 | 80.6 | 38438 | 477 |
| 15 | 20 | .053 | 4.7 | 22.1 | 92 | 1000 | 76.3 | 45563 | 597 |
| 16 | 20 | .048 | 4.7 | 22.0 | 100 | 1000 | 79.9 | 43350 | 543 |
| 17 | 20 | .050 | 4.6 | 21.9 | 100 | 1000 | 82.5 | 42375 | 514 |
| 18 | 20 | .067 | 4.7 | 21.6 | 92 | 1000 | 83.3 | 51150 | 614 |
| 19 | 20 | .030 | 4.7 | 21.6 | 100 | 1000 | 76.4 | 34650 | 454 |
| 20 | 20 | .077 | 4.6 | 21.7 | 0 | 1000 | 94.7 | 21375 | 226 |
| 21 | 30 | .048 | 4.9 | 21.9 | 59 | 1000 | 107.2 | 59550 | 556 |
| 22 | 30 | .043 | 4.7 | 21.7 | 100 | 1000 | 108.9 | 54563 | 501 |
| 23 | 30 | .040 | 4.6 | 21.8 | 100 | 1000 | 103.9 | 62100 | 598 |

17

## TABLE 4 (cont.)

| DAY | % Glucose in Feed | HR RT | pH | T | %DO | RPM | DRY CELL g/l | ASTAXANTHIN | |
|-----|-------------------|-------|-----|------|-----|------|------|------|------|
| | | | | | | | | μ/l | PPM |
| 24 | 30 | .037 | 4.8 | 21.6 | 100 | 1000 | 96.1 | 42450 | 442 |
| 25 | 30 | .053 | 4.6 | 21.8 | 41 | 1000 | 107.1 | 62625 | 585 |
| 26 | 30 | .043 | 4.6 | 21.8 | 35 | 1000 | 94.3 | 81188 | 861 |
| 27 | 30 | .056 | 4.6 | 21.8 | 35 | 1000 | 99.0 | 54938 | 555 |
| 28 | 30 | .048 | 4.6 | 22.0 | 39 | 1000 | 128.3 | 88875 | 693 |
| 29 | 30 | .056 | 4.6 | 21.8 | 47 | 1000 | 119.7 | 66000 | 551 |
| 30 | 30 | .053 | 4.6 | 22.0 | 39 | 1000 | 106.7 | 74250 | 696 |
| 31 | 30 | .037 | 4.6 | 21.8 | 56 | 1000 | 104.5 | 76875 | 736 |

INOCULUM MEDIA = 100 ML YEPD

STARTER FERMENTER MEDIA = 1M3 + 2% GLUCOSE + .1% YEAST EXTRACT + BIOTIN 0.08 mg/l + THIAMINE 1.00 mg/l

10% FEED = FM21 + 10% GLUCOSE + .2% YEAST EXTRACT + BIOTIN 0.08 mg/l + THIAMINE 1.00 mg/l

20% FEED = 2x FM21 + 20% GLUCOSE + .4% YEAST EXTRACT + BIOTIN 0.08 mg/l + THIAMINE 1.00 mg/l

0% FEED = 3x FM21 + 30% GLUCOSE + .6% YEAST EXTRACT + BIOTIN 1.60 mg/l + THIAMINE 2.00mg/l

ND = Not determined

B. Batch-Fed Growth of *Phaffia rhodozyma* in 4 Liter Fermentor

This example provides the protocol used to grow strains of *Phaffia rhodozyma* in a 4 liter fermentation vessel in a batch-fed process.

A fresh culture of *Phaffia rhodozyma* was innoculated into a 250 ml Erlemmeyer flask containing 100 ml of shake flask media (shake flask media comprising 0.6% yeast extract, 0.6% malt extract, and 2.0% glucose). The flask was then incubated at about 20°C for 72 hours. The contents were transferred to a 2800 ml Fernbach flask containing 1000 ml of shake flask media and again incubated at about 20°C for 24 hours. The contents were then transferred to the 4 liter fermenter containing 1000 ml of 2x BioLaffitte media (twice as concentrated). The initial fermentor volume was 2 liters with pH = 5.2 and 20°C.

During the 4 day run, pH was controlled between 5.2 and 5.5 (using ammonia and phosphoric acid), the temperature was controlled between 19°C and 21°C (using a cooling water bath), and dissolved oxygen (DO) was maintained between 30% and 80% saturation (by increasing agitation and airflow if necessary).

The fermentation was run so as to maintain a slight positive glucose (0.1% to 0.3%) for the first 50 hours. After depletion of the original glucose in the fermentor, the feed was started from a 50% glucose reservoir (750g glucose + 750 g $H_2O$, sterile). The initial feed rates were generally slow, but increased rapidly thereafter. Glucose concentration was monitored by HPLC during this process. The fermentation was then allowed to run glucose limited (glucose not detectable). The fermentation was complete 24 hours after all the feed was added, provided there is no detectable glucose in the broth. To control foaming 3-4 drops of MAZU DF37C Mazer Chemicals, Gurnee, Ilinois, was added per liter of working volume as needed.

## C. Batch-Fed Growth of *Phaffia rhodozyma* in 20 Liter Vessel

This example provides the protocol used for growing *Phaffia rhodozyma* in a 20 liter fermentation vessel in a batch-fed process.

A fresh culture of *Phaffia rhodozyma* was innoculated into a 250 ml Erlemmeyer flask containing 100 ml of shake flask media (shake flask media comprising 0.6% yeast extract, 0.6% malt extract, and 2.0% glucose). The flask was then incubated at about 20°C for 72 hours. The contents were transferred to a 2800 ml Fernbach flask containing 1000 ml of shake flask media and again incubated at about 20°C for 24 hours. The contents were then transferred to the BioLafitteTM fermenter containing 9 liters of Biolaffitte media. The initial fermentor volume was 10 liters with pH = 5.2 and 20°C.

During the 4 day run, pH was controlled between 5.2 and 5.5 (using ammonia and phosphoric acid), the temperature was controlled between 19°C and 21°C (using a cooling water bath), and dissolved oxygen (DO) was maintained between 30% and 80% saturation (by increasing agitation, pressure, and air flow if necessary).

The fermentation was run so as to maintain a slight positive glucose (0.1% to 0.3%) for the first 50 hours. After depletion of the original glucose in the fermentor, the feed was started from a 50% glucose reservoir (3750g glucose + 3750 g $H_2O$, sterile). The initial feed rates were generally slow, but increased rapidly thereafter. Glucose concentration was monitored by HPLC during this process. The fermentation was then allowed to run glucose limited (glucose not detectable). The fermentation was complete 24 hours addition of all the feed, provided there is no detectable glucose in the broth. To control foaming 3-4 drops of MAZU DF37C Mazer Chemicals, Gurnee, Ilinois, was added per liter of working volume as needed.

## D. Novel Strains

The following tables demonstrate the high productivities of produced in the novel strains of the present invention.

TABLE 5

| Mutant | Plate[1] | Astaxanthin µg/g | |
| | | Shake Flask[2] | Fermentor[3] |
| --- | --- | --- | --- |
| PC8059 | 877 | 726 | 1108 |
| PC8065 | 559 | 720 | 900 |
| PC8088 | 1622 | 1462 | ND |
| PC8091 | 1988 | 3531 | 3300 |
| PC8093 | 1811 | 3570 | 1121 |
| PC8071 | 977 | 1792 | ND |
| PC8075 | 1681 | 2179 | 2155 |
| PC8077 | 1345 | 2602 | 2009 |

[1]The plates were grown and assayed as described in Example I.C.1.

[2]The shake flasks cultures were grown on 50 ml Rich Assay Growth Medium in a 250 ml Ehrlenmeyer flask from a loop full of a *Phaffia rhodozyma* culture (taken from a 7 day old plate culture). The inoculated 250 ml flask was incubated at 20-22°C with vigorous shaking. The strains were grown for 5 days (120 hours) then harvested and assayed.

[3]The fermentor cultures were grown as described in Example II, B or C.

TABLE 6

| Mutant | Astaxanthin µg/l/hr | |
| | Shake Flask[1] | Fermentor[2] |
| --- | --- | --- |
| PC8059 | 78.9 | 405 |
| PC8065 | 55.9 | 480 |
| PC8088 | 80.7 | ND |
| PC8091 | 51.5 | 961 |
| PC8093 | 55.0 | 401 |
| PC8071 | 57.5 | ND |
| PC8075 | 97.7 | 415 |
| PC8077 | 47.0 | 269 |

[1]The shake flasks cultures were grown as described in Table 5.

[2]The fermentor cultures were grown as described in Example II, B or C.

Example III

A. Enzymatic Lysis of *Phaffia rhodozyma*

This example demonstrates that the digestive enzyme preparation from *Trichoderma harzianium* is very effective in releasing the astaxanthin contain in *Phaffia rhodozyma* cells.

The following enzymes were used to lyse the *Phaffia rhodozyma* cells from strain PC 8059 (NRRL Y-18549) in order to release the astaxanthin. All were tested at 30°C and manufacturer's recommended pH. The results are summarized in Table 7. The cell concentration prior to lysis was approximately 68 g/l.

The *Phaffia rhodozyma* cells utilized in this example were grown in as described in Example II.C 2. The percentage of astaxanthin released was determined by photometrically measuring the absorption (at 478 mm) of each sample after treatment with each enzyme and dividing that value by the absorption value obtained by acetone extraction of similarly grown *Phaffia rhodozyma* cells. Equal quantities of cells were used in both the enzyme treatment and the acetone extraction. The acetone extraction was performed as described in Example I.C.2.b.

## Table 7

| ENZYME | DOSAGE | TIME | ASTAXANTHIN RELEASED* |
|---|---|---|---|
| NOVO MUTANASE SP299[1] (powder) | 0.1 g/1 | 24 HR | 79% |
| | | 48 HR | 92% |
| | 0.12 g/1 | 22 HR | 81% |
| | | 43 HR | 95% |
| | 0.5 g/1 | 24 HR | 99% |
| | 1 g/1 | 24 HR | 99% |
| | 1 g/1 | 24 HR | 97% |
| GENENCOR CYTOLASE 123[2] (liquid) | 10 ml/1 | 27 HR | 38% |
| | | 48 HR | 60% |
| | | 120 HR | 62% |
| | 10 ml/1 | 48 HR | 29% |
| | 50 ml/1 | 48 HR | 55% |
| NOVO VISCOZYME 120L[3] (liquid) | 10 ml/1 | 24 HR | 22% |
| | | 48 HR | 28% |
| NOVO GAMANASE 1.5L[4] (liquid) | 10 ml/1 | 24 HR | 5% |
| NOVO CEREMIX 2XL[5] (liquid) | 10 ml/1 | 24 HR | 8% |

\* As determined in Example I.C.2.

[1]Mutanase is manufactured by Novo Laboratories of Danbury, CT.

[2]Cytolase 123 is manufactured by Genencor of South San Francisco, CA.

[3]Viscozyme is manufactured by Novo Laboratories of Danbury, CT.

[4]Gamanase is manufactured by Novo Laboratories of Danbury, CT.

[5]Ceremix is manufactured by Novo Laboratories of Danbury, CT.

The results demonstrate that MutanaseTM, a digestive enzyme preparation from *Trichoderma harzianium* is particularly effective in lysing *Phaffia* cells compared to the other enzymes preparations tested.

B. Mechanical Breakage of *Phaffia rhodozyma*

Samples of *Phaffia rhodozyma* PC 8057 produced in Example II A on day 31 were subjected to mechanical cell breakage using a bead mill, Gaulin press, and a Microfluidics microfluidizer. The results are summarized in Table B.

## Table 8

### Mechanical Breakage of *Phaffia rhodozyma* strain?

| Pass | Percent Astaxanthin Extractable[1] | | |
| --- | --- | --- | --- |
| | Bead Mill[2] | Gaulin Press[3] | Micro Fluidics[4] |
| 0 | 12 | 7 | 15 |
| 1 | 32 | 75 | 73 |
| 2 | 37 | 92 | 93 |
| 3 | 40 | 99 | 101 |
| 4 | 42 | 98 | 102 |
| 5 | | 99 | 101 |
| Control[1] | 100 | 100 | 100 |

[1]based on acetone extractable astaxanthin from cells disrupted 6 min in bead beater (7.875 pg astaxanthin/ml of cell suspension).

[2]Manufactured by Willy A. Bachofen Machinenfabrik, Basel, Switzerland, Dyno-Mill.

[3]Provided by Gaulin Corporation, Everett, Ma., model 65 M3 10TBSX

Cell Homogenizer Model 110T operated with a flow rate of 4 liters/minute with a 20 liter reseroir at 9500 psi provided by Microfluidic Corporation, Newton, Ma.

Example IV

This example demonstrates the use of astaxanthin derived from *Phaffia rhodozyma* is an effective dietary pigment supplement for salmonoids. This example also shows that effective preparation of *Phaffia rhodozyma* cells is necessary before the astaxanthin contained in the *Phaffia rhodozyma* cells will be available to salmonoids as a source of dietary pigment.

A. Growth of *Phaffia rhodozyma*

The *Phaffia rhodozyma* strain used in this example was PC 8059. One loop each of PC 8059 culture was transferred from a 7 day old plate to 4 separate 250 ml Erlenmeyer flasks containing 100 mls of modified YM broth (without agar). The 250 ml Erlenmeyer flasks were incubated on an oval shaker (250 rpm) for 48 hours at 22° C. 50 mls from each flask were separately withdrawn and transferred to 4 2.8 liter Fernbach flasks containing 1 liter of modified YM broth (without agar). The Fernbach flasks were incubated in a rotary shaker (250 rpm) for 56 hours at 22° C.

The 4 liters of inoculant from the Fernbach were transferred into a 400 liter fermentor containing 156 liters of FM-21 salts containing 2% Amberex™ 1003 (see Table 2). The 400 liter fermentor was equiped with monitors and controls for pH, dissolved oxygen, agitator speed, temperature, air flow, pressure, foam, and weight. The pH in the fermentor was adjusted to 4.8 with ammonia. Feed addition was started at 0.6 liters/hour and glucose concentration was monitored by HPLC. Feed rate was steadily increased after inoculation to the maximum rate of 2.7 to 2.8 liters/hour, when feed addition was completed. A total of 125 liters of feed was added during the runs. During feeding the fermentor was held at 22°C with pH controlled between 4.8 and 5.5. The dissolved oxygen content was maintained between 30% and 60% of saturation. After the feed was consumed the fermentor was held for 24 hours at a temperature of 22°C and the pH was allowed to drift up to 6.4. The dissolved oxygen content during the whole period was controlled at less than 90% by reducing air flow and agitation speed.

The first 400 liter fermentor run ended with a total volume of 275 liters. 150 liters of this run were homogenized and spray dried. The remaining 125 liters was spray dried without homogenization to be used as whole cells. A second 400 liter fermentor as described above was performed resulting in a final volume of 200 liters. The 200 liters from this run were enzymatically broken and spray dried.

Spray drying was performed on 125 to 150 liter batches of fermentation broth. 400 ppm antioxidant ethoxyquin, 1,000 ppm Rangen Vitamin C Poly-phosphate, and 1,000 ppm ascorbytylpalmitate were added to each batch of fermentor broth. The broth was then pasturized at 75°C to 86°C. The pasturized fermentation broth was then dried in a bench top spray drier with an inlet temperature of 280°C to 284°C and an outlet of 80°C to 96°C.

Homogenization of the *Phaffia* cells from the fermentation broth was performed with 150 liter batches of broth which had been held in a 300 liter tank at 10°C. Homogenization was performed with a Gaulin homogenizer at 8,000 psig to 10,000 psig in recycle mode with a flow rate of 4 liters per unit for a total of 4 hours (equivalent to 6.4 passes through the homogenizer).

Enzymatic digestion of *Phaffia rhodozyma* was performed using 26 grams of Novo Mutanase™ SP299 (Novo Laboratories, Danbury, Conneticut). The 26 grams of Novo Mutanase™ SP299 was dissolved in 200 mls of deionized water and filter sterilized before addition to the fermentation broth. The dissolved Mutanase™ was then added to 220 liters of broth in the 400 fermentor. The broth was then incubated with gentle agitation (100 rpm on the agitator) under a nitrogen blanket at 30°C for 22 hours. The astaxanthin content of each of these samples was determined by HPLC as described in Example 2. The trout feed utilized in the following feeding studies was formulated with an objective of obtaining equal concentrations of total astaxanthin in the trout feed.

B. Trout Feeding Studies - Feed and Time Comparisons

Table 9 below describes the results of trout feeding studies utilizing Carophyll Pink, a negative control, mechanically broken cells, whole cells, and enzymatically broken cells of *Phaffia rhodozyma*. This table demonstrates that mechanically broken *Phaffia rhodozyma* cells or enzymatically broken *Phaffia rhodozyma* cells provide substantial availability of all the astaxanthin present in the *Phaffia rhodozyma* cells. Table 9 also demonstrates that unbroken *Phaffia rhodozyma* cells will not provide significant amounts of astaxanthin as a dietary pigment supplement. As shown by Table 9, the extractability of astaxanthin determined by acetone extraction correlates well with the utilization of astaxanthin contained in *Phaffia rhodozyma* cells by salmonoids. The details of this study are set forth below.

EP 0 454 024 A2

## Table 9

### Astaxanthin Content of Trout

| Diet | 30 days[a] | 60 days[b] | 90 days[b] |
|---|---|---|---|
| **DIET 1** | | | |
| Trout Flesh (ppm) | 5.01 ± 1.89 | 5.62 ± 1.58 | 6.65 ± 3.31 |
| Feed (ppm) | 72.2 | 53.3 | 53.3 |
| **DIET 2** | | | |
| Trout Flesh (ppm) | 0 | 0 | 0 |
| Feed (ppm) | 0 | 0 | 0 |
| **DIET 3** | | | |
| Trout Flesh (ppm) | 1.57 ± 0.92 | 2.89 ± 0.80 | 5.47 ± 2.05 |
| Feed (ppm) | 30.8 | 31.8 | 31.8 |
| **DIET 4** | | | |
| Trout Flesh (ppm) | 0.59 ± 0.34 | 0.90 ± 0.47 | 1.68 ± 0.93 |
| Feed (ppm) | 9.6 | 8.8 | 8.8 |
| **DIET 5** | | | |
| Trout Flesh (ppm) | 1.89 ± 0.93 | 2.48 ± 1.41 | 5.38 ± 1.67 |
| Feed (ppm) | 32.7 | 35.0 | 35.0 |

Diet 1:  Carophyll Pink
Diet 2:  Negative Control
Diet 3:  Mechanically Broken *Phaffia*
Diet 4:  Whole Cell *Phaffia*
Comment: (Diet 4, Feed (ppm) is available astaxanthin as determined by acetone extraction.  Total Astaxanthin is about 31 ppm for each period).
Diet 5:  Enzymatically Broken *Phaffia*
[a] Diet in Table 10
[b] Diet in Table 11

Trout feeding studies were conducted for 90 days as outlined above. The trout diet at 30 days is defined in Table 10. This diet was reformulated at 60 days to the diet defined in Table 11, which was used for the remainder of the feeding studies. Product A (shown in Tables 10 and 11) was used in Diet 3 and contained mechanically broken *Phaffia rhodozyma* cells (strain PC8059), Diet 3 contained 30-32 ppm available astaxanthin. Product B (shown in Tables 10 and 11) was used in Diet 4 and contained whole *Phaffia rhodozyma* cells (strain PC8059), Diet 4 contained 29 ppm astaxanthin total astaxanthin. Product C (shown in Tables 10 and 11) was used in Diet 5 and contained enzymatically broken (using Mutanase™) *Phaffia rhodozyma* cells (strain PC8059) Diet 5 contained 30 ppm astaxanthin. All *Phaffia rhodozyma* cells were grown in the 400 l fermenter as described in Example II.

24

## TABLE 10

### Ingredients of test Feeds 1-5 fed to rainbow trout in *Phaffia rhodozyma* yeast feeding trial.

| Ingredients | Diet 1 (%) | Diet 2 (%) | Diets 3-5 (%) |
|---|---|---|---|
| Herring meal | 39.00 | 39.00 | 39.00 |
| Soybean meal | 14.00 | 14.00 | 9.00 |
| [1]Products A, B, or C | 0.00 | 0.00 | 5.00 |
| Cottonseed meal | 10.00 | 10.00 | 10.00 |
| Blood meal | 5.00 | 5.00 | 5.00 |
| Brewer's yeast | 5.00 | 5.00 | 5.00 |
| Wheat clears | 8.55 | 8.55 | 8.55 |
| Dried Whey | 5.00 | 5.00 | 5.00 |
| [2]Permapel | 2.00 | 2.00 | 2.00 |
| Fish oil | 9.60 | 9.60 | 9.60 |
| [3]Vitamin mix | 0.40 | 0.40 | 0.40 |
| [4]Mineral mix | 0.10 | 0.10 | 0.10 |
| Choline Chloride (70%) | 0.10 | 0.10 | 0.10 |
| [5]STAY-C | 0.11 | 0.11 | 0.11 |
| Gelatin | 1.40 | 1.14 | 1.14 |
| [6]CP | 0.10 | 0.00 | 0.00 |
| Total | 100.00 | 100.00 | 100.00 |

[1]Spray-dried *Phaffia rhodyozyma*

[2]Binder

[3]US F&WS No. 30, see list of media, buffers and solutions at the beginning of the Examples.

[4]US F&WS No. 3, see list of media, buffers and solutions at the beginning of the Examples.

[5]L-ascorbyl-2-polyphosphate (100 ppm Ascorbic Acid-equivalent, w/w)

[6]Carophyll Pink 5% Astaxanthin (minimum) in gelatin carrier (Hoffman La-Roche)

## TABLE 11

### Ingredients of test Feeds 1-5 fed to rainbow trout in *Phaffia rhodozyma* yeast feeding trial.

| Ingredients | Diet 1 (%) | Diet 2 (%) | Diets 3-5 (%) |
|---|---|---|---|
| Anchovy meal | 30.00 | 30.00 | 30.00 |
| Soybean meal | 14.00 | 14.00 | 14.00 |
| [1]Products A, B, or C | 0.00 | 0.00 | 5.00 |
| Cottonseed meal | 10.00 | 10.00 | 10.00 |
| Blood meal | 5.00 | 5.00 | 5.00 |
| Brewer's yeast | 5.00 | 5.00 | 5.00 |
| Wheat clears | 17.55 | 17.55 | 17.55 |
| Dried Whey | 5.00 | 5.00 | 5.00 |
| [2]Permapel | 2.00 | 2.00 | 2.00 |
| Fish oil | 9.60 | 9.60 | 9.60 |
| [3]Vitamin mix | 0.40 | 0.40 | 0.40 |
| [4]Mineral mix | 0.10 | 0.10 | 0.10 |
| Choline Chloride (70%) | 0.10 | 0.10 | 0.10 |
| [5]STAY-C | 0.11 | 0.11 | 0.11 |
| Gelatin | 1.10 | 1.10 | 1.10 |
| [6]CP | 0.08 | 0.00 | 0.00 |
| Total | 100.00 | 100.00 | 100.00 |

[1]Spray-dried *Phaffia rhodyozyma*

[2]Binder

[3]US F&WS No. 30, see list of media, buffers and solutions at beginning of Examples.

[4]US F&WS No. 3, see list of media, buffers and solutions at beginning of Examples.

[5]L-ascorbyl-2-polyphosphate (100 ppm Ascorbic Acid-equivalent, w/w)

[6]Carophyll Pink 5% Astaxanthin (minimum) in gelatin carrier (Hoffman La-Roche)

## C. Extraction of Pigment from Trout Feed Pre-mix and Rations

2 g of each of the 5 feed pre-mixes were weighed out in duplicate and placed in 40 ml polypropylene centrifuge tubes. Approximately 5 g of each of the 5 trout ration pellets were ground to a fine powder in a morter and pestle. 2 g of each of the 5 ground trout ration pellets were weighed out in duplicate and placed in 40 ml polypropylene centrifuge tubes.

10 ml of deionized water was added to each tube from the pre-mix and trout ration samples. The tubes were incubated at 55°C for 10 min with occasional shaking and allowed to cool to room temperature. 20 ml of acetone was added to each centrifuge tube and vortexed for 30 seconds. The solids were pelleted by centrifugation at 12,000 xg for 5 min at 22°C. The supernatant was decanted and retained from each tube.

20 ml of acetone was then added to the supernatant and vortexed for 30 sec. The solids were again pelleted by centrifugation at 12,000 xg for 5 min at 22°C. The supernatants were decanted and cooled for each extract.

10 ml of methylene chloride ($CH_2Cl_2$) were added to each sample (20 ml were added to samples containing Carophyll Pink) and vortexed. The phases were allowed to separate. The lower phase containing pigment was removed and retained. A second $CH_2Cl_2$ extraction was performed and the pigment containing fractions were cooled. Solvent was removed from each sample by weathering to dryness under a nitrogen purge in reduced light (in the hood) at room temperature.

Each sample was redissolved in 10.0 ml of n-hexane and 1 g of anhydrous $Na_2SO_4$ was added to remove any trace of water. 1.0 ml of each sample was then loaded onto a Florisil Cartridge Sep Pak (water to remove lipids from the sample). This was washed with 10 ml of n-hexane followed by 10 ml of 70:30 n-hexane:diethyl ether. Any pigment bound to the column was backflushed with 10 ml of acetone and retained. Any acetone remaining was removed from each sample again by weathering to dryness under a nitrogen purge in reduced light (in the hood) at room temperature. Each sample was redissolved in 1.0 ml of methanol, and total astaxanthin concentration was determined as in Example I.C.2.

D. Extraction of Pigment from Rainbow Trout Flesh

About 10 g of rainbow trout flesh was minced with a razor blade (the exact weight was recorded). 20 ml of acetone was added and the sample was ground in a morter and pestle. The acetone extract was decanted into a disposable plastic centrifuge tube and 10 ml of acetone was again added to the sample. The sample was ground again and sample and acetone were transferred to the same centrifuge tube. The sample was centrifuged at 2000 rpm for 10 min at 20°C. The supernatant was decanted into an 11 dram glass vial and dried under a $N_2$ stream. 10 ml of acetone was again added to the plastic centrifuge tubes, vortexed, and centrifuged at 2000 rpm for 10 min at 20°C. The supernatant was again decanted into the 11 dram glass vial. (At this stage, if the pellet is not colorless, this extraction should be repeated). The sample was again dried under a $N_2$ stream until only the aqueous phase was left.

1 ml of diethylether and 5 ml of n-hexane were added to the extract and vortexed vigorously. The phases were allowed to separate, and the upper organic phase (containing pigment) was removed to a glass vial using a Pasteur pette and dried under a $N_2$ stream.

The sample was again extracted with 5 ml of n-hexane, the phases were allowed to separate, and the upper organic phase was again removed. This extraction procedure was repeated until the upper organic phase was colorless. The samples were pooled and dried under a $N_2$ stream. The pigment was redissolved in 10 ml of n-hexane and a pinch of anhydrous $Na_2SO_4$ was added to remove any trace of water.

3.0 ml of each extract was loaded onto a Silica Cartridge Sep Pak. This was washed with 10 ml of n-hexane followed by 5 ml of 70:30 n-hexane:diethylether. Any pigment bound to the column was backflushed with 10 ml of acetone and retained. Acetone was removed from each sample by drying under a $N_2$ stream. Each sample was redissolved in 3.0 ml methanol and astaxanthin concentrations were determined as in Example I.C.2.

**Claims**

1. A strain of Phaffia rhodozyma which produces in the range of 45 to 100 $\mu$g/l/hr of astaxanthin on a dry weight basis when cultivated in a shake flask.

2. The strain of claim 1 which produces in the range of 55 to 100 $\mu$g/l/hr of astaxanthin on a dry weight basis when cultivated in a shake flask.

3. The strain of claim 1 which produces in the range of 78 to 100 $\mu$g/l/hr of astaxanthin on a dry weight basis when cultivated in a shake flask.

4. The strain of Phaffia rhodozyma which produces in the range of 400 to 962 $\mu$g/l/hr of astaxanthin on a dry weight basis when cultivated in a batch-fed fermentation vessel.

5. The strain of claim 4 which produces from 480 to 962 $\mu$g/l/hr of astaxanthin on a dry weight basis when cultivated in a batch-fed fermentation vessel.

6. Phaffia rhodozyma strain deposited as NRRL Y-18549, NRRL Y-18550, NRRL Y-18554, NRRL Y-18555, NRRL Y-18556, NRRL Y-18551, NRRL Y-18552 and NRRL Y-18553.

7. A method for the growth of Phaffia rhodozyma cells which produce high yields of astaxanthin comprising:

27

(a) contacting Phaffia rhodozyma cells with a nutrient media to form a fermentation broth; and
(b) cultivating said fermentation broth in the presence of a measurable excess of at least one carbon-energy source wherein said excess is supplied in an amount not resulting in the production of a growth inhibiting amount of alcohol or aldehyde.

8. The method of claim 7 wherein during said cultivation the pH is maintained in the range of 3.0 to 5.5.

9. The method of claim 7 or 8 wherein during said cultivation the temperature is maintained in the range of 18 to 22 °C.

10. The method of any of claims 7-9 wherein the Phaffia rhodozyma is grown in a batch-fed manner and the aeration condition is maintained with a dissolved oxygen content in the range of 10 to 80 % saturation.

11. The method of any of claims 7-10 wherein the measurable excess of said carbon-energy source is in the range of 1 to 20 g/l based on the fermentation broth volume.

12. A method for the growth of Phaffia rhodozyma cells which produce high yields of astaxanthin comprising:
(a) contacting Phaffia rhodozyma cells with a nutrient media comprising nitrogen and phosphate sources, minerals and trace elements to form a fermentation broth;
(b) maintaining said fermentation broth at a pH from 5.2 to 5.4, at a temperature in the range of 18 to 22 °C and at a dissolved oxygen content of 30 to 60 % saturation, and
(c) contacting the fermentation broth during cultivation with at least one carbon-energy source and maintaining a measurable excess of said carbon-energy source ranging from 1 to 5 g/l in said fermentation broth, wherein said carbon-energy source is supplied in an amount which will not result in the production of a growth inhibiting amount of alcohol or aldehyde.

13. The method of any of claims 7-12 wherein said carbon-energy source is selected from succinate, furamate, malate, pyruvate, glucose, sucrose, fructose, maltose, corn syrup, hydrolyzed starch, and combinations of any two or more thereof.

14. The method of any of claims 7-12 wherein the carbon-energy source is selected from succinate, glucose and combinations thereof.

15. A method which provides Phaffia rhodozyma cells containing astaxanthin in a form wherein the astaxanthin is available to salmonids, crustaceans and birds as dietary source of astaxanthin said method comprising contacting Phaffia rhodozyma cells containing astaxanthin with a digestive enzyme preparation from the fungus Trichoderma harzianum capable of at least partially digesting the cell wall of Phaffia rhodozyme.

16. The method of claims 15 wherein said digestive enzyme preparation is used in an amount from 0.2 to 10.0 units per 100 g/l.

17. The method of claims 15 or 16 being carried out at a temperature from 0 to 60 °C, preferably 20 to 30 °C, and a pH ranging from 4.0 to 5.5, preferable from 4.5 to 5.